# FASCICULE DE BREVET EUROPEEN

(11) **EP 2 793 946 B1**
(45) Date de publication et mention de la délivrance du brevet: **06.03.2019**
(21) Numéro de dépôt: 11818946.3
(22) Date de dépôt: 20.12.2011
(51) Int. Cl.: A61K 47/36, A61K 9/00, A61K 8/49, A61Q 3/02

(54) **COMPOSITION TOPIQUE FILMOGÈNE ET SON UTILISATION POUR TRAITER OU PRÉVENIR L'ONYCHOPHAGIE**
TOPISCHE FILMBILDENDEN ZUSAMMENSETZUNG UND IHRE VERWENDUNG ZUR BEHANDLUNG ODER PRÄVENTION VON ONYCHOPHAGIE
TOPICAL FILM-FORMING COMPOSITION, AND USE THEREOF FOR TREATING OR PREVENTING ONYCHOPHAGIA

(43) Date de publication de la demande: 29.10.2014
(73) Titulaire: Urgo Recherche Innovation et Développement, 21300 Chenove (FR)
(72) Inventeur: BOUVIER, Claire, F-21000 Dijon (FR)
(74) Mandataire: Cabinet Plasseraud
(86) Numéro de dépôt international: PCT/FR2011/053070
(87) Numéro de publication internationale: WO 2013/093216

(56) Documents cités:
- EP-A1- 1 958 638
- FR-A1- 2 773 067
- JP-A- 2001 114 651

## Description

La présente invention a pour objet une composition topique filmogène comprenant au moins du benzoate de dénatonium et un chitosane, dérivé de chitosane, ou un sel pharmaceutiquement acceptable de ceux-ci, ainsi que son utilisation pour traiter ou prévenir l'onychophagie.

L'onychophagie est le terme médical désignant l'habitude que présentent certaines personnes de se ronger les ongles. Particulièrement fréquente durant l'enfance et l'adolescence, elle se retrouve néanmoins dans toutes les tranches d'âge.

On peut distinguer deux types d'onychophagie, légère ou sévère, suivant l'intensité des dégâts causés aux ongles, aux doigts et aux dents.

L'onychophagie légère consiste à se ronger les ongles seulement. L'onychophagie sévère quant à elle se caractérise par des saignements, des blessures, et des lésions qui peuvent conduire à des infections répétées.

L'onychophagie est un comportement inconscient qui démarre souvent à l'enfance et a tendance à s'arrêter spontanément.

Des études ont mis en avant que des facteurs de stress, d'anxiété, de solitude, ou d'ennui constituaient des causes récurrentes de l'onychophagie.

Il est courant de prévenir l'onychophagie par l'application sur les ongles de compositions filmogènes présentant un goût particulièrement amer. Le benzoate de dénatonium est un composé bien connu et couramment utilisé pour son goût amer prononcé dans les compositions de vernis à ongles développées pour traiter ou prévenir l'onychophagie.

Le benzoate de dénatonium présente l'inconvénient de ne pas conférer une bonne tenue dans le temps de l'amertume. En effet, dès 12 heures, le goût amer est significativement atténué et l'application de la composition de traitement des ongles doit, la plupart du temps, être renouvelée régulièrement pour une bonne efficacité.

Il existe donc un besoin pour une composition filmogène de traitement ou de prévention de l'onychophagie, présentant une amertume durable dans le temps, notamment plus de 12 heures, voire au-delà de 24h.

La demanderesse a montré, de manière surprenante, qu'il était possible d'améliorer la tenue dans le temps de l'amertume conférée par le benzoate de dénatonium en le formulant en association avec un chitosane, dérivé de chitosane, ou un sel pharmaceutiquement acceptable de ceux-ci.

L'invention a donc pour objet, selon un premier aspect, une composition topique filmogène comprenant, dans un milieu pharmaceutiquement acceptable, au moins du benzoate de dénatonium et un chitosane, dérivé de chitosane, ou un sel pharmaceutiquement acceptable de ceux-ci, selon la revendication 1.

On entend par « composition topique » au sens de la présente demande, une forme d'administration d'une composition sur une région déterminée du corps.

Selon l'invention, la composition topique peut être une solution, une crème, une pommade, un onguent, un gel, une lotion, une mousse, ou un vernis, et de préférence un vernis à ongles. L'invention concerne également l'utilisation d'une telle composition pour traiter ou prévenir l'onychophagie.

Bien entendu, la composition selon l'invention ne traite pas, à proprement parler, une quelconque maladie à l'origine de l'onychophagie, mais prévient la manie développée par certains sujets de se ronger les ongles et s'intéresse uniquement au traitement de ce symptôme de l'onychophagie. Il en résulte que l'utilisation de la composition selon l'invention pour traiter ou prévenir l'onychophagie est non thérapeutique.

### Benzoate de dénatonium

La composition filmogène selon l'invention comprend au moins du benzoate de dénatonium.

Le benzoate de dénatonium est un sel benzoïque de dénatonium au goût très amer. Il est communément utilisé comme dénaturant, agent répulsif, agent d'aversion ou amérisant.

Dès 1960, le benzoate de dénatonium, sous la marque Bitrex®, est autorisé aux États-Unis et au Royaume-Uni dans les parfums, la parfumerie, les cosmétiques et autres usages industriels. Depuis, il est reconnu comme un agent dénaturant et agent d'aversion dans plus de 40 pays. En 1993, il est autorisé en Europe comme dénaturant de l'alcool pour décourager la consommation des alcools toxiques tels que le méthanol et l'éthylène glycol.

Le benzoate de dénatonium est un sel artificiel comprenant un anion (le benzoate) associé à un cation (un ammonium quaternaire) et présente la formule (I) suivante :

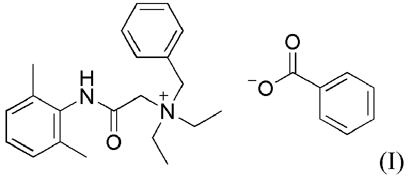

Le dénatonium peut être associé avec d'autres anions que le benzoate sous forme de sel tels que l'ion saccharinate pour former le saccharinate de dénatonium ou l'ion chlorure pour former le chlorure de dénatonium, mais sa forme pharmaceutiquement acceptable la plus amère reste le sel benzoïque.

Dans le cadre de la présente invention, le benzoate de dénatonium utilisé peut par exemple être le produit Bitrex®, commercialisé par la société Macfarlan Smith.

Le benzoate de dénatonium est présent dans la composition filmogène en une teneur suffisante pour conférer un goût amer à la composition, en une teneur allant de 0,001 à 15% en poids, par rapport au poids total de la composition, de préférence de 0,05 à 10% en poids, et plus préférentiellement de 0,5 à 6% en poids.

### Chitosane ou dérivé de chitosane

La composition filmogène selon l'invention comprend, en outre, au moins un chitosane, dérivé de chitosane, ou un sel pharmaceutiquement acceptable de ceux-ci.

Le chitosane est un polysaccharide composé de la distribution aléatoire de D-glucosamine liée en β-(1-4) (unité désacétylée) : 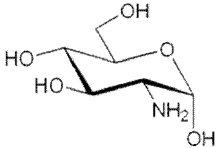 et de N-acétyl-D-glucosamine (unité acétylée) :

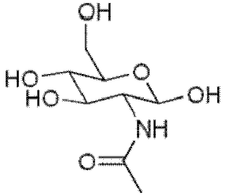

Il est produit par désacétylation chimique (en milieu alcalin) ou enzymatique de la chitine, composant de l'exosquelette des arthropodes (crustacés) ou de l'endosquelette des céphalopodes (calamars...) ou encore de la paroi des champignons.

Cette matière première est déminéralisée par traitement à l'acide chlorhydrique, puis déprotéinée en présence de soude ou de potasse et enfin décolorée grâce à un agent oxydant.

Le chitosane présente ainsi la structure suivante :

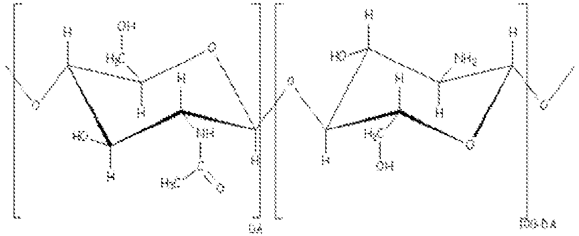

Le degré d'acétylation (DA) est le pourcentage d'unités acétylées par rapport au nombre d'unités totales, il peut être déterminé par spectrométrie infrarouge à transformée de Fourier (IR-TF) ou par un titrage par une base forte.

La différence entre chitosane et chitine correspond à un degré d'acétylation de 50% : en deçà, le composé est nommé chitosane, au-delà, chitine.

Le degré d'acétylation (DA) se distingue du degré de déacétylation (DD), l'un étant l'inverse de l'autre. Ainsi, un chitosane ayant un degré de déacétylation de 85%, possède 15% de groupements acétyles et 85% de groupements amines sur ses chaînes.

Les dérivés de chitosane sont notamment choisis parmi les hydroxyalkylchitosanes et les carboxyalkylchitosanes, de préférence solubles dans l'eau, et présentant notamment un poids moléculaire supérieur à 5 000 g/mol, et de préférence compris entre 10 000 et 50 000 g/mol. Selon un mode particulièrement préféré de réalisation, le dérivé de chitosane est l'hydroxypropylchitosane.

En effet, la demanderesse a observé que la tenue dans le temps de l'amertume conférée par le benzoate de dénatonium était particulièrement améliorée lorsque celui-ci est associé avec l'hydroxypropylchitosane.

En particulier, le dérivé de chitosane, et de préférence l'hydroxypropylchitosane, est présent dans la composition filmogène en une teneur allant de 0,01 à 10% en poids, par rapport au poids total de la composition, de préférence de 0,1 à 3% en poids, et plus préférentiellement de 0,5 à 1% en poids.

Selon un mode de réalisation particulier de l'invention, le benzoate de dénatonium et le dérivé de chitosane, de préférence l'hydroxypropylchitosane, sont présents dans la composition filmogène selon l'invention en un rapport pondéral de benzoate de dénatonium:hydroxypropylchitosane allant de 1:10000 à 1500:1, de préférence de 12:1 à 2:1.

### Milieu pharmaceutiquement acceptable

La composition selon l'invention comprend un milieu pharmaceutiquement acceptable comprenant au moins de l'eau et/ou un solvant organique.

Par milieu pharmaceutiquement acceptable, on entend, au sens de la présente demande, un milieu compatible avec les matières kératiniques, notamment la peau et les phanères.

L'eau peut, par exemple, être présente dans la composition selon l'invention en une teneur allant de 0,1 à 30% en poids, par rapport au poids total de la composition, de préférence de 5 à 20% en poids.

Le solvant organique peut notamment être choisi parmi :
- les cétones liquides à température ambiante tels que méthyléthylcétone, méthylisobutylcétone, diisobutylcétone, l'isophorone, la cyclohexanone, l'acétone ;
- les alcools, autres que les diols vicinaux précédemment décrits, liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyl éther de propylène glycol, le mono n-butyl éther de dipropylène glycol ;
- les éthers cycliques tels que la γ-butyrolactone ;
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle ;
- les éthers tels que le diéthyléther, le diméthyléther ou le dichlorodiéthyléther ;
- les alcanes liquides à température ambiante tels que le décane, l'heptane, le dodécane, le cyclohexane ;
- les alkyl sulfoxides tels que le diméthylsulfoxide ;
- les aldéhydes liquides à température ambiante tels que le benzaldéhyde, l'acétaldéhyde ;
- le 3-éthoxypropionate d'éthyle ;
- les carbonates tels que le carbonate de propylène, le carbonate de diméthyle,
- les acétals tels que méthylal ;
et leurs mélanges.

Selon un mode préféré de réalisation, le solvant organique est choisi parmi:
- les alcools, autres que les diols vicinaux précédemment décrits, liquides à température ambiante tels que l'éthanol, l'isopropanol, le diacétone alcool, le 2-butoxyéthanol, le cyclohexanol, et de préférence l'éthanol,
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle, et de préférence l'acétate d'éthyle,
et leurs mélanges.

Selon un mode plus préféré de réalisation, le solvant organique utilisé dans les compositions selon l'invention est constitué d'un mélange d'éthanol et d'acétate d'éthyle.

Le solvant organique peut représenter de 40 à 98% en poids, de préférence de 60 à 95% en poids, et plus préférentiellement de 70 à 90% en poids, par rapport au poids total de la composition.

Selon un mode particulièrement préféré de réalisation, le milieu pharmaceutiquement acceptable utilisé dans les compositions selon l'invention est constitué d'un mélange d'eau, d'éthanol et d'acétate d'éthyle.

### Polymère filmogène

La composition comprend, avantageusement, au moins un polymère filmogène autre que le chitosane ou dérivé de chitosane précédemment décrit.

Selon la présente invention, on entend par "polymère filmogène", un polymère apte à former à lui seul ou en présence d'un agent auxiliaire de filmification, un film continu et adhérent sur un support, notamment sur la peau ou les phanères.

Parmi les polymères filmogènes utilisables dans la composition de la présente invention, on peut citer les polymères synthétiques, de type radicalaire ou de type polycondensat, les polymères d'origine naturelle et leurs mélanges.

Le polymère filmogène peut être choisi en particulier parmi les polymères cellulosiques tels que la nitrocellulose, l'acétate de cellulose, l'acétobutyrate de cellulose, l'acétopropionate de cellulose, l'éthylcellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide, le copolymère de PolyVinylMéthyl éther et de Maleic Anhydride (PVM/MA).

Comme polymère filmogène, on peut notamment utiliser la nitrocellulose RS 1/8 sec ; RS [1/4] sec. ; [1/2] sec. ; RS 5 sec. ; RS 15 sec. ; RS 35 sec. ; RS 75 sec.; RS 150 sec ; AS [1/4] sec. ; AS [1/2] sec. ; SS [1/4] sec. ; SS [1/2] sec. ; SS 5 sec., notamment commercialisée par la société HERCULES, ou la nitrocellulose DHL 120/170 IPA commercialisée par la société NOBEL ; les résine toluène sulfonamide formaldéhyde "Ketjentflex MS80" de la société AKZO ou "Santolite MHP", "Santolite MS 80" de la société FACONNIER ou "RESIMPOL 80" de la société PAN AMERICANA, la résine alkyde "BECKOSOL ODE 230-70-E" de la société DAINIPPON, la résine acrylique "ACRYLOID B66" de la société ROHM & HAAS, la résine polyuréthane "TRIXENE PR 4127" de la société BAXENDEN.

Selon un mode préféré de réalisation de la présente demande, le polymère filmogène utilisé dans la composition est l'éthylcellulose, notamment commercialisée par la société Herculès sous la dénomination commerciale EC-N100 Pharm.

Le polymère filmogène peut être présent dans la composition selon l'invention en une teneur en matières sèches allant de 0,1 % à 15 % en poids, de préférence allant de 1 % à 10 % en poids, par rapport au poids total de la composition.

### Agent auxiliaire de filmification

Pour améliorer les propriétés filmogènes de la composition, un agent auxiliaire de filmification peut avantageusement être ajouté.

L'agent auxiliaire de filmification est, bien évidemment, différent du solvant organique présent dans le milieu pharmaceutiquement acceptable.

Un tel agent auxiliaire de filmification peut être choisi parmi tous les composés connus de l'homme du métier, comme étant susceptibles de remplir la fonction recherchée, et être notamment choisi parmi les agents plastifiants et les agents de coalescence du ou des polymère(s) filmogène(s).

Ainsi, la composition peut comprendre, en outre, au moins un agent plastifiant et/ou un agent de coalescence. En particulier, on peut citer, seuls ou en mélange, les plastifiants et agents de coalescence usuels, tels que :
- les alcools gras comme l'octyldodécanol, le 2-butyloctanol, le 2-hexyl décanol, le 2-undécylpentadécanol, l'alcool oléique ;
- les glycols et leurs dérivés, tels que la glycérine, le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les acides gras tels que l'acide oléique, l'acide linoléique, l'acide linolénique ;
- les esters de glycol tels que la triacétine (ou triacétate de glycéryle) ;
- les dérivés de propylène glycol et en particulier le propylène glycol phényléther, le propylène glycol diacétate, le dipropylène glycol éthyléther, le tripropylène glycol méthyléther, le propylène glycol butyléther ;
- les esters d'acides, notamment carboxyliques, tels que les citrates, les phtalates, les adipates, les carbonates, les tartrates, les phosphates, les sébacates et en particulier les esters d'acide monocarboxylique tels que l'isononanoate d'isononyle, l'érucate d'oléyle ou le néopentanoate d'octyl-2-docécyle ;
- les dérivés oxyéthylénés, tels que les huiles oxyéthylénées, notamment les huiles végétales, telles que l'huile de sésame, l'huile de ricin, l'huile d'amande, l'huile de canola, l'huile de noisette, l'huile de pistache, l'huile de lin, l'huile de bourrache, l'huile de chanvre, l'huile de jojoba, l'huile de tournesol, l'huile de germe de blé, l'huile de maïs et/ou de germe de maïs, l'huile d'arachide, l'huile d'avocat, l'huile de carthame, l'huile de colza, l'huile d'olive, l'huile d'argan, l'huile de tournesol, l'huile de pépin de raisin, l'huile de soja, l'huile de noix, l'huile de pépin de de courge, l'huile de palme, l'huile de coprah, et leurs mélanges. L'huile peut également être un dérivé d'une des huiles végétales citées précédemment. Il peut s'agir d'huile hydrogénée ou non, peroxydée ou non.
et leurs mélanges.

Selon un mode préféré de réalisation, l'agent auxiliaire de filmification est choisi parmi les dérivés oxyéthylénés, tels que les huiles oxyéthylénées, notamment les huiles végétales, telles que l'huile de ricin.

Selon un mode plus préféré de réalisation, l'agent auxiliaire de filmification est l'huile de ricin.

Par exemple, la teneur en agent auxiliaire de filmification peut aller de 0,01 % à 10% en poids, et en particulier de 0,5 % à 5 % en poids, par rapport au poids total de la composition.

### Additifs

La composition selon l'invention peut comprendre un ou plusieurs additifs pharmaceutiquement acceptables, comme par exemple les parfums, les arômes, les colorants, les pigments, les agents matifiants, les agents rhéologiques, les conservateurs, les vitamines, les huiles essentielles et les agents actifs, notamment choisis parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques et les filtres solaires.

En particulier, les actifs pouvant être introduits dans la composition selon l'invention peuvent être choisis parmi :
- les anti-bactériens tels que le Polymyxine B, les pénicillines (Amoxycilline), l'acide clavulanique, les tétracyclines, la Minocycline, la chlorotétracycline, les aminoglycosides, l'Amikacine, la Gentamicine, la Néomycine, l'argent et ses sels (Sulfadiazine argentique), les probiotiques ;
- les antiseptiques tels que le mercurothiolate de sodium, l'éosine, la chlorhexidine, le borate de phénylmercure, l'eau oxygénée, la liqueur de Dakin, le triclosan, le biguanide, l'hexamidine, le thymol, le Lugol, la Povidone iodée, le Merbromine, le Chlorure de Benzalkonium et de Benzethonium, l'éthanol, l'isopropanol ;
- les anti-viraux tels que l'Aciclovir, le Famciclovir, le Ritonavir ;
- les antifongiques tels que les polyènes, le Nystatin, l'Amphotéricine B, la Natamycine, les imidazolés (Miconazole, Ketoconazole, Clotrimazole, Éconazole, Bifonazole, Butoconazole, Fenticonazole, Isoconazole, Oxiconazole, Sertaconazole, Sulconazole, Thiabendazole, Tioconazole), les triazolés (Fluconazole, Itraconazole, Ravuconazole, Posaconazole, Voriconazole), les allylamines, la Terbinafine, l'Amorolfine, la Naftifine, la Buténafine ;
- la Flucytosine (antimétabolite), la Griséofulvine, la Caspofungine, la Micafungine ;
- les anti-douleurs tels que le Paracétamol, la Codéine, le Dextropropoxyphène, le Tramadol, la Morphine et ses dérivés, les Corticoïdes et dérivés ;
- les anti-inflammatoires tels que les Glucocorticoïdes, les anti inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique ;
- les actifs favorisant la cicatrisation tels que le Rétinol, la Vitamine A, la Vitamine E, la N-acétyl-hydroxyproline, les extraits de Centella Asiatica, la papaïne, les silicones, les huiles essentielles de thym, de niaouli, de romarin et de sauge, l'acide hyaluronique, les oligosaccharides polysulfatés synthétiques ayant 1 à 4 unités oses tels que le sel de potassium du sucrose octasulfaté, le sel d'argent du sucrose octasulfaté ou le sucralfate, l'Allantoïne ;
- les agents hydratants tels que l'acide hyaluronique, l'urée, le glycérol, les acides gras, modulateurs des aquaporines, les huiles végétales, certains sucres dont le sorbitol, les beurres et les cires ;
- les agents dépigmentants tels que l'acide kojique (Kojic Acid SL® - Quimasso (Sino Lion)), l'Arbutine (Olevatin® - Quimasso (Sino Lion)), le mélange de palmitoylpropyl de sodium et d'extrait de nénuphar blanc (Sepicalm® - Seppic), l'undécylénoyl phénylalanine (Sepiwhite® - Seppic), l'extrait de réglisse obtenue par fermentation d'Aspergillus et éthoxydiglycol (Gatuline Whitening® - Gattefossé), l'acide octadécènedioïque (ODA White® - Sederma), l'alpha-arbutin (Alpha-arbutin®, SACI-CFPA (Pentapharm)), l'extrait aqueux de feuilles Arctophylos Uva Ursi (Melfade-J® - SACI-CFPA (Pentapharm)), le mélange de plante complexe Gigawhite® (SACI-CFPA (Alpaflor)), la diacétyl boldine (Lumiskin® - Sederma), l'extrait de mandarine du Japon (Melaslow® - Sederma), le mélange d'extrait de citron enrichi en acide citrique et d'extrait de concombre (Uninontan®U-34 - Unipex), le mélange d'extrait de Rumex occidentalis et de vitamine C (Tyrostat® 11 - Unipex), des oligopeptides (Mélanostatine 5® - Unipex), le dipalmitate kojique (KAD-15® - Quimasso (Sino Lion)), le complexe d'origine naturelle Vegewhite® de LCW, des extraits de germe de blé (Clariskin® II - Silab), l'éthyldiamine triacétate (EDTA);
- les agents kératolytiques tels que l'acide salicylique, le salicylate de zinc, l'acide ascorbique, les acides alpha hydroxylés (acide glycolique, lactique, malique, citrique, tartrique), les extraits d'Erable argenté, de Griottier, de Tamarinier, l'urée, le rétinoïde topique Kératoline® (Sederma), les protéases obtenues par fermentation de Bacillus Subtilis, le produit Linked-Papain® (SACI-CFPA), la papaïne (enzyme protéolytique issue du fruit de papaye);
- les actifs restructurants (par exemple restructurant des phanères) tels que les dérivés de silice, la vitamine E, la camomille, le calcium, l'extrait de prêle, le Lipester de soie;
- les anesthésiques tels que la benzocaïne, la lidocaïne, la dibucaïne, le chlorhydrate de pramoxine, la bupivacaïne, la mepivacaïne, la prilocaïne, l'étidocaïne ;
- les filtres solaires, tels que les filtres chimiques (Oxybenzone, Sulisobenzone, Dioxybenzone, Tinosorb S®, Avobenzone, p-méthoxycinnamate de 2-éthoxyéthyle, Uvinul® A+, Mexoryl® XL, Méthoxycinnamate ou octinoxate d'octyle, Salicylate ou octisalate d'octyle, octyl triazone ou Uvinul® T 150, salicylate de méthyle, meradimate, enzacamène, MBBT ou Tinosorb® M, cyanophénylcinnamate d'octyle ou Parsol® 340, Acide para-aminobenzoïque, Ensulizole, Parsol® SLX ou Polysiloxane-15 ou Benzylidène malonate polysiloxane, salicylate de triéthanolamine ou salicylate de trolamine, Mexoryl® SX ou acide téréphtalylidène dicamphosulfonique) et les filtres minéraux (oxydes de Zinc, dioxyde de titane, kaolin, ichtyol)
et leurs mélanges.

Selon un mode préféré de réalisation, la composition selon l'invention comprend au moins un actif restructurant et notamment la vitamine E (tocophérol) ou l'acétate de vitamine E (acétate de tocophérol).

### Utilisation de la composition

Selon un mode particulier de réalisation, l'invention a pour objet l'utilisation non thérapeutique de la composition telle que définie précédemment pour traiter ou prévenir l'onychophagie.

La composition selon l'invention présente une tolérance fonctionnelle excellente lorsqu'elle est appliquée sur des sujets pendant une durée prolongée, et permet d'améliorer l'aspect général des ongles et de les renforcer. Les ongles sont ainsi moins cassants et moins striés.

L'invention a également pour objet, selon un autre aspect, l'utilisation de chitosane, dérivé de chitosane, ou un sel pharmaceutiquement acceptable de ceux-ci, et de préférence l'utilisation d'hydroxypropylchitosane pour améliorer la tenue dans le temps de l'amertume conférée par une composition filmogène comprenant du benzoate de dénatonium.

La composition selon l'invention présente ainsi un goût amer pendant au moins 12h, et de préférence au moins 24h après application.

La présente invention est illustrée plus en détails dans les exemples non limitatifs décrits ci-après.

### EXEMPLE

On a préparé une composition filmogène de vernis à ongle selon l'invention (Composition 1) comprenant les constituants suivants:

| Composition 1 | % en poids |
|---|---|
| Ethylcellulose | 3.0 |
| Acétate d'éthyle | 10.0 |
| Ethanol absolu | 63,5 |
| Bitrex (benzoate de dénatonium) | 4.0 |
| Huile de ricin | 1.0 |
| Acétate de tocophérol | 0.5 |
| Hydroxypropylchitosane | 0.8 |
| Eau distillée | 17,2 |

La composition a été préparée en mélangeant l'éthanol, l'eau distillée et l'hydroxypropylchitosan.

Après 5 minutes, l'acétate d'éthyle et le Bitrex® ont été incorporés au mélange.

Une fois que l'ensemble des constituants ont été solubilisés, l'huile de ricin et l'acétate de tocophérol, puis l'éthylcellulose ont été ajoutés.

L'ensemble de ces étapes se sont déroulées sous agitateur à hélice jusqu'à dissolution complète des constituants.

On a préparé une composition filmogène comparative (Composition comparative 2) analogue à la composition 1 sans hydroxypropyle chitosane (balance faite sur l'éthanol).

Dès 12h après l'application sur ongles, il a été observé que le goût amer de la composition comparative 2 était sensiblement atténué, alors que l'amertume de la composition 1 selon l'invention était encore bien présente après 24h.

### Test consommateurs :

20 personnes présentant des ongles sains, mais rongés, ont été sélectionnées pour tester la composition selon l'invention et ont démarré le protocole à.

Une fois par jour, chaque personne a appliqué le vernis selon l'invention le matin après la douche pendant 28 jours.

La population étudiée était composée de 4 hommes et 16 femmes d'un âge moyen de 42 ans (± 15 ans) compris entre 18 et 66 ans.

### Tolérance :

Après 28 jours d'utilisation du produit, le dermatologue n'a relevé aucun signe d'intolérance cutanée et la tolérance fonctionnelle a été excellente.

### Efficacité :

Le vernis a été jugé efficace par :
- 95% des sujets pour empêcher de se ronger les ongles,
- 90% sur l'amélioration de l'aspect général des ongles,
- 80% sur le renforcement des ongles,
- 75% sur les ongles moins cassants,
- 45% sur les ongles moins striés.

En outre, 100% des sujets ont jugé que le vernis présentait un temps de séchage rapide (moins de 2 minutes), qu'il était facile d'utilisation et discret.

95% des sujets ont jugé l'odeur du vernis une fois sec neutre.

### Tenue dans le temps de l'amertume :

95% des sujets ont jugé que l'amertume tenait pendant 12h et 85% pendant 24h.

## Revendications

1. Vernis à ongles filmogène comprenant, dans un milieu pharmaceutiquement acceptable, au moins du benzoate de dénatonium et un chitosane, dérivé de chitosane, ou un sel pharmaceutiquement acceptable de ceux-ci ;
ledit chitosane, dérivé de chitosane, ou un sel pharmaceutiquement acceptable de ceux-ci étant présent dans la composition filmogène en une teneur allant de 0,01 à 10% en poids, par rapport au poids total de la composition de vernis à ongles, et
ledit benzoate de dénatonium étant présent dans la composition filmogène en une teneur allant de 0,001 à 15% en poids, par rapport au poids total de la composition de vernis à ongles.

2. Vernis à ongles selon la revendication 1, **caractérisé en ce que** le dérivé de chitosane est choisi parmi les hydroxyalkylchitosanes et les carboxyalkylchitosanes.

3. Vernis à ongles selon l'une des revendications précédentes, **caractérisé en ce que** le dérivé de chitosane est soluble dans l'eau, et présente un poids moléculaire supérieur à 5 000 g/mol, et de préférence compris entre 10 000 et 50 000 g/mol.

4. Vernis à ongles selon l'une des revendications précédente, **caractérisée en ce que** le dérivé de chitosane est l'hydroxypropylchitosane.

5. Vernis à ongles selon l'une des revendications précédente, **caractérisée en ce que** le dérivé de chitosane, et notamment l'hydroxypropylchitosane, est présent dans la composition filmogène en une teneur allant de 0,1 à 3% en poids, par rapport au poids total de la composition de vernis à ongles, plus préférentiellement de 0,5 à 1% en poids.

6. Vernis à ongles selon l'une des revendications précédentes, **caractérisé en ce que** le benzoate de dénatonium est présent dans la composition filmogène en une teneur allant de 0,05 à 10% en poids, par rapport au poids total de la composition de vernis à ongles, plus préférentiellement de 0,5 à 6% en poids.

7. Vernis à ongles selon l'une quelconque des revendications précédentes, **caractérisé en ce que** le benzoate de dénatonium et le dérivé de chitosane, de préférence l'hydroxypropylchitosane, sont présents dans le vernis à ongles filmogène selon l'invention en un rapport pondéral de benzoate de dénatonium:hydroxypropylchitosane allant de 1:10000 à 1500:1, de préférence de 12:1 à 2:1.

8. Vernis à ongles selon l'une quelconque des revendications précédentes, **caractérisé en ce que** le milieu pharmaceutiquement acceptable comprend au moins de l'eau et/ou un solvant organique choisi parmi:
- les alcools liquides à température ambiante tels que l'éthanol, l'isopropanol, le diacétone alcool, le 2-butoxyéthanol, le cyclohexanol, et de préférence l'éthanol,
- les esters à chaîne courte (ayant de 3 à 8 atomes de carbone au total) tels l'acétate d'éthyle, l'acétate de butyle, l'acétate de méthyle, l'acétate de propyle, l'acétate d'isopropyle, l'acétate d'isopentyle, l'acétate de méthoxypropyle, le lactate de butyle, et de préférence l'acétate d'éthyle,
et leurs mélanges.

9. Vernis à ongles selon la revendication 8, **caractérisé en ce que** l'alcool est choisi parmi les mono-alcools, par exemple l'éthanol, les glycols, par exemple le caprylyl glycol, et leurs mélanges.

10. Vernis à ongles selon l'une quelconque des revendications 8 ou 9, **caractérisé en ce que** le solvant organique est constitué d'un mélange d'éthanol et d'acétate d'éthyle.

11. Vernis à ongles selon l'une quelconque des revendications précédentes, **caractérisé en ce que** le milieu pharmaceutiquement acceptable est constitué d'un mélange d'eau, d'éthanol et d'acétate d'éthyle.

12. Vernis à ongles selon l'une quelconque des revendications précédentes, **caractérisé en ce qu'**il comprend un polymère filmogène, de préférence l'éthylcellulose.

13. Vernis à ongles selon l'une quelconque des revendications précédentes, **caractérisé en ce qu'**il comprend un agent auxiliaire de filmification choisi parmi les dérivés oxyéthylénés, tels que les huiles oxyéthylénées, notamment les huiles végétales, telles que l'huile de ricin.

14. Vernis à ongles selon l'une quelconque des revendications précédentes, **caractérisé en ce qu'**il comprend un ingrédient choisi parmi les agents anti-bactériens, les antiseptiques, les anti-viraux, les agents antifongiques, les anti-douleurs, les anti-inflammatoires, les agents favorisant la cicatrisation, les agents hydratants, les agents dépigmentants, les agents kératolytiques, les actifs restructurants, les anesthésiques, les filtres solaires et leurs mélanges, et de préférence un actif restructurant, notamment la vitamine E (tocophérol) ou l'acétate de vitamine E (acétate de tocophérol).

15. Utilisation non thérapeutique d'un vernis à ongles filmogène selon l'une quelconque des revendications précédentes, pour traiter ou prévenir l'onychophagie.

## Patentansprüche

1. Filmbildender Nagellack, welcher in einem pharmazeutisch akzeptablen Medium wenigstens ein Denatoniumbenzoat und ein Chitosan, Chitosanderivat oder ein pharmazeutisch akzeptables Salz der letzteren umfasst;
wobei das Chitosan, Chitosanderivat oder ein pharmazeutisch akzeptables Salz der letzteren in der filmbildenden Zusammensetzung mit einem Gehalt von 0,01 bis 10 Gewichtsprozent in Bezug auf das Gesamtgewicht der Nagellackzusammensetzung vorliegt, und
wobei das Denatoniumbenzoat in der filmbildenden Zusammensetzung mit einem Gehalt von 0,001 bis 15 Gewichtsprozent in Bezug auf das Gesamtgewicht der Nagellackzusammensetzung vorliegt.

2. Nagellack nach Anspruch 1, **dadurch gekennzeichnet, dass** das Chitosanderivat gewählt ist aus Hydroxyalkylchitosanen und Carboxyalkylchitosanen.

3. Nagellack nach einem der vorhergehenden Ansprüche, **dadurch gekennzeichnet, dass** das Chitosanderivat wasserlöslich ist und ein Molekulargewicht größer 5 000 g/mol, und vorzugsweise von 10 000 bis 50 000 g/mol aufweist.

4. Nagellack nach einem der vorhergehenden Ansprüche, **dadurch gekennzeichnet, dass** das Chitosanderivat Hydroxypropylchitosan ist.

5. Nagellack nach einem der vorhergehenden Ansprüche, **dadurch gekennzeichnet, dass** das Chitosanderivat und insbesondere das Hydroxypropylchitosan, in der filmbildenden Zusammensetzung mit einem Gehalt von 0,1 bis 3 Gewichtsprozent in Bezug auf das Gesamtgewicht der Nagellackzusammensetzung vorliegt, stärker bevorzugt mit 0,5 bis 1 Gewichtsprozent.

6. Nagellack nach einem der vorhergehenden Ansprüche, **dadurch gekennzeichnet, dass** das Denatoniumbenzoat in der filmbildenden Zusammensetzung mit einem Gehalt von 0,05 bis 10 Gewichtsprozent in Bezug auf das Gesamtgewicht der Nagellackzusammensetzung vorliegt, stärker bevorzugt mit 0,5 bis 6 Gewichtsprozent.

7. Nagellack nach einem der vorhergehenden Ansprüche, **dadurch gekennzeichnet, dass** das Denatoniumbenzoat und das Chitosanderivat, vorzugsweise das Hydroxypropylchitosan, in der erfindungsgemäßen filmbildenden Zusammensetzung mit einem Gewichtsverhältnis von Denatoniumbenzoat:Hydroxypropylchitosan von 1:10000 bis 1500:1, vorzugsweise von 12:1 bis 2:1 vorliegen.

8. Nagellack nach einem der vorhergehenden Ansprüche, **dadurch gekennzeichnet, dass** das pharmazeutisch akzeptable Medium wenigstens Wasser und/oder ein organisches Lösungsmittel umfasst, welches gewählt ist aus:
- bei Raumtemperatur flüssigen Alkoholen wie Ethanol, Isopropanol, Diacetonalkohol, 2-Butoxyethanol, Cyclohexanol und bevorzugt Ethanol,
- kurzkettige Ester (mit insgesamt 3 bis 8 Kohlenstoffatomen) wie Ethylacetat, Butylacetat, Methylacetat, Propylacetat, Isopropylacetat, Isopentylacetat, Methoxypropylacetat, Butyllactat und vorzugsweise Ethylacetat, und deren Mischungen.

9. Nagellack nach Anspruch 8, **dadurch gekennzeichnet, dass** der Alkohol gewählt ist aus Mono-Alkoholen wie beispielsweise Ethanol, und Glycole wie beispielsweise Caprylylglycol, und deren Mischungen.

10. Nagellack nach einem der Ansprüche 8 oder 9, **dadurch gekennzeichnet, dass** das organische Lösungsmittel eine Mischung aus Ethanol und Ethylacetat umfasst.

11. Nagellack nach einem der vorhergehenden Ansprüche, **dadurch gekennzeichnet, dass** das das pharmazeutisch akzeptable Medium eine Mischung aus Wasser, Ethanol und Ethylacetat umfasst.

12. Nagellack nach einem der vorhergehenden Ansprüche, **dadurch gekennzeichnet, dass** er ein filmbildendes Polymer, vorzugsweise Ethylcellulose aufweist.

13. Nagellack nach einem der vorhergehenden Ansprüche, **dadurch gekennzeichnet, dass** er ein Filmbildungshilfsmittel umfasst, welches gewählt ist aus Oxyethylenderivaten, wie beispielsweise Oxyethylen-Öle, insbesondere pflanzliche Öle, wie beispielsweise Ricinusöl.

14. Nagellack nach einem der vorhergehenden Ansprüche, **dadurch gekennzeichnet, dass** er einen Bestandteil umfasst, welcher gewählt ist aus antibakteriellen Mitteln, antiseptischen Mitteln, antiviralen Mitteln, antimykotischen Mitteln, Schmerzmitteln, entzündungshemmenden Mitteln, wundheilungsfördernden Mitteln, hydratisierenden Mitteln, Depigmentierungsmitteln, keratolytischen Mitteln, restrukturierenden Wirkstoffen, Anästhetika, Sonnenschutzmitteln und deren Mischungen, und bevorzugt einen restrukturierenden Wirkstoff, insbesondere Vitamin E (Tocopherol) oder Vitamin-E-Acetat (Tocopherolacetat).

15. Nicht therapeutische Anwendung eines filmbildenden Nagellacks nach einem der vorhergehenden Ansprüche zur Behandlung oder Prävention der Onychophagie.

## Claims

1. Film-forming nail polish comprising, in a pharmaceutically acceptable medium, at least denatonium benzoate and a chitosan, chitosan derivative, or a pharmaceutically acceptable salt thereof;
said chitosan, chitosan derivative, or a pharmaceutically acceptable salt thereof being present in the film-forming composition in a content ranging from 0.01 to 10% by weight, based on the total weight of the nail polish composition, and
said denatonium benzoate being present in the film-forming composition in a content ranging from 0.001 to 15% by weight, based on the total weight of the nail polish composition.

2. Nail polish according to claim 1, **characterised in that** the chitosan derivative is chosen from hydroxyalkylchitosans and carboxyalkylchitosans.

3. Nail polish according to one of the preceding claims, **characterised in that** the chitosan derivative is soluble in water, and has a molecular weight greater than 5,000 g/mol, and preferably between 10,000 and 50,000 g/mol.

4. Nail polish according to one of the preceding claims, **characterised in that** the chitosan derivative is hydroxypropylchitosan.

5. Nail polish according to one of the preceding claims, **characterised in that** the chitosan derivative, and in particular hydroxypropylchitosan, is present in the film-forming composition in a content ranging from 0.1 to 3% by weight, based on the total weight of the nail polish composition, more preferably from 0.5 to 1% by weight.

6. Nail polish according to one of the preceding claims, **characterised in that** the denatonium benzoate is present in the film-forming composition in a content ranging from 0.05 to 10% by weight, based on the total weight of the nail polish composition, more preferably from 0.5 to 6% by weight.

7. Nail polish according to any preceding claim, **characterised in that** the denatonium benzoate and the chitosan derivative, preferably hydroxypropylchitosan, are present in the film-forming nail polish according to the invention in a weight ratio of denatonium benzoate:hydroxypropylchitosan ranging from 1:10,000 to 1,500:1, preferably from 12:1 to 2:1.

8. Nail polish according to any preceding claim, **characterised in that** the pharmaceutically acceptable medium comprises at least water and/or an organic solvent chosen from:
- liquid alcohols at ambient temperature such as ethanol, isopropanol, diacetone alcohol, 2-butoxyethanol, cyclohexanol, and more preferably ethanol,
- short-chain esters (having from 3 to 8 carbon atoms in total) such as ethyl acetate, butyl acetate, methyl acetate, propyl acetate, isopropyl acetate, isopentyl acetate, methoxypropyl acetate, butyl lactate, and more preferably ethyl acetate,
and mixtures thereof.

9. Nail polish according to claim 8, **characterised in that** the alcohol is chosen from mono-alcohols, for example ethanol, glycols, for example caprylyl glycol, and mixtures thereof.

10. Nail polish according to any of claims 8 or 9, **characterised in that** the organic solvent is comprised of a mixture of ethanol and ethyl acetate.

11. Nail polish according to any preceding claim, **characterised in that** the pharmaceutically acceptable medium is comprised of a mixture of water, ethanol and ethyl acetate.

12. Nail polish according to any preceding claim, **characterised in that** it comprises a film-forming polymer, preferably ethylcellulose.

13. Nail polish according to any preceding claim, **characterised in that** it comprises an auxiliary film-forming agent chosen from oxyethylenated derivatives, such as oxyethylenated oils, in particular vegetable oils, such as castor oil.

14. Nail polish according to any preceding claim, **characterised in that** it comprises an ingredient chosen from anti-bacterial agents, antiseptics, anti-viral agents, antifungal agents, pain-killers, anti-inflammatory drugs, agents promoting the healing of wounds, moisturising agents, depigmenting agents, keratolytic agents, restructuring agents, anaesthetics, sunscreens and mixtures thereof, and more preferably a restructuring agent, in particular vitamin E (tocopherol) or vitamin E acetate (tocopherol acetate).

15. Non-therapeutic use of a film-forming nail polish according to any preceding claim, for treating or preventing onychophagia.
